# EUROPEAN PATENT APPLICATION

(11) **EP 4 803 492 A1**
(43) Date of publication of application: **09.09.2026**
(21) Application number: 25161739.5
(22) Date of filing: 05.03.2025
(51) Int. Cl.: C02F 3/34, C02F 101/38, C02F 103/36

(54) **A MIXTURE OF MICROORGANISMS THAT DECOMPOSES AMINES FOUND IN REFINERY WASTEWATER**

(71) Applicant: Star Rafineri Anonim Sirketi, 34396 Istanbul (TR)
(72) Inventor: ALPAY UYANIKER, Tayyibe, 35800 Izmir (TR); PASHAYEVA, Bike, 35800 Izmir (TR); SARIKAYA, Pelin, 35040 Izmir (TR); OZDEMIR, Guven, 35040 Izmir (TR); EZDESIR, Ayhan, 35800 Izmir (TR)
(74) Representative: Sevinç, Cenk

(57) **Abstract**

The invention relates to a bacterial culture mixture that eliminates nitrogen from wastewater in order to biodegrade amine compounds found in refinery wastewater and the method of obtaining this mixture. In the bacterial culture mixture that is the subject of the invention, pure bacterial isolates of the genera *Arthrobacter, Pseudomonas, Pigmentiphaga, Acidovorax, Chitinophaga* obtained as pure isolates from refinery wastewater activated sludge are multiplied in bioreactor systems, in suitable mineral medium, trace element solution and bacterial growth media. With said bacterial culture mixture, the treatment of water is ensured by degrading 2-aminoethanol, 2-diethylaminoethanol and piperazine in wastewater.

## Description

### Technical Field of the Invention

The invention relates to a bacterial culture mixture that eliminates nitrogen from wastewater in order to biodegrade amine compounds, 2-aminoethanol, 2-diethylaminoethanol and piperazine, found in refinery wastewater, and the method of obtaining this mixture. The biological treatment of wastewater is provided with said bacterial culture mixture.

### State of the Art

Wastewater is defined as used water affected by domestic, industrial and commercial use, however, the composition of all wastewater constantly varies depending on the area of use. Although the components in the content of wastewater vary, wastewater contains toxic elements for humans and the ecosystem. For this reason, the main aim of wastewater treatment is to remove as much of the suspended solids as possible from the remaining water, called waste, before it is discharged back into the environment. If these waters are not treated appropriately, the environment and human health are adversely affected. Excessive amounts of phosphorus and nitrogen (including ammonia) in wastewater can cause eutrophication or excessive fertilisation in water, which can be toxic to aquatic organisms. In addition, the presence of bacteria, viruses and any pathogens that cause disease in said waters poses a danger to aquatic organisms, wildlife and humans [1]. Unclean water poses significant health risks, causing 1.7 million deaths annually, more than 90 percent of which occur in developing countries [2].

The main source of wastewater from industrial use is refineries. Refineries are among the major consumers of water due to their cooling towers and process uses, therefore, large amounts of wastewater are generated during the treatment and refinement of crude oil, which requires treatment. The wastewater produced contains significant amounts of ammonia and toxic decomposition products, nitroamines and nitrosamines [3]. Both pose major threats to the ecological environment, especially the overstimulation of the growth of aquatic plants and algae, which can result in blockage of water intakes, reduction of dissolved oxygen concentrations reaching living beings, prevention of light reaching deeper waters [4], and to human health, particularly upper respiratory tract diseases in humans, reduced oxygenation of body tissues, fluid accumulation in the lungs, and death. In order to prevent these problems, the most common method used in the state of the art to remove nitrogen compounds from wastewater released into the environment as a result of industrial applications is biological nitrogen removal (BNR). Biological nitrogen removal technologies are widely used to remove nitrogen from wastewater and protect natural water quality. In conventional wastewater treatment plants, ammonium is first oxidised to nitrite, which is then oxidised to nitrate under aerobic conditions. The nitrate is then reduced to nitrogen gas by a nitrogen remover using electrons donated by organic matter. This process requires a significant amount of energy to provide oxygen for the consumption of ammonium oxidising bacteria (AOB) and nitrite oxidising bacteria (NOB) and organic matter for denitrification. Organic matter is an important energy source due to its usability for methane production by anaerobic methanogenic bacteria, and to increase nitrogen removal, most organic matter is used as a carbon source for denitrification. This results in lower energy production by methanogenic bacteria. Therefore, autotrophic nitrogen removal technology, which can remove nitrogen without using organic matter, is stated to be a more sustainable way for wastewater treatment [5]. However, some problems are encountered during the BNR process, including the slow growth of microorganisms involved in biological processes and the control of simultaneous nitrification/denitrification conditions. As a solution to these problems, shortcut biological nitrogen removal (SBNR) has been intensively studied in recent years. This process is based on the fact that nitrite is an intermediate compound in both nitrification and denitrification. Nitrite, which is formed after the ammonium oxidation stage and is an intermediate compound, is directly reduced to nitrogen gas by heterotrophic denitrifying bacteria under anoxic conditions. For the activation of the shortcut biological nitrogen removal process, the amount of AOB must be increased and NOBs must be inhibited. Another method used in wastewater treatment is activated sludge systems, which were first introduced in 1914 and are still used today. The activated sludge process is a method designed to accelerate the rate of decomposition of waste material in water and functions as a multi-compartment reactor unit that uses high concentrations of microorganisms to break down organics and remove nutrients from wastewater, producing quality wastewater [6]. This system is generally effective for easily degradable compounds of wastewater. Considering that many industrial wastes may contain man-made chemicals that are foreign to microorganisms and resistant to decomposition by microorganisms, great difficulties can be seen during the use of activated sludge systems in the biological treatment of such wastes. In particular, the sudden entry of recalcitrant compounds, that is, compounds that are generally not easily degraded microbiologically, into these systems or the increase in their proportions in the inlet water causes the system to fail. This system failure is due to the decrease in the bacterial population capable of degrading these recalcitrant compounds. In addition, both nitrogen removal and complex organic matter removal cannot be provided at the same time, which requires more than one treatment step and treatment times are extended. In addition, due to the resistance of bacteria that can metabolise recalcitrant compounds to harsh and toxic environments, there are also problems such as system overload, toxic substances or inability to maintain stability even in changing environmental conditions.

The patent document numbered CN105255754A in the state of the art describes a bacterium for degrading ammonia nitrogen and total nitrogen, its application and a processing method. Said bacterium (*Pseudomonas sp.* ZXY-1) is separated from contaminated soil in Jilin Chemical's pesticide factory. It is mentioned that the bacterium can use ammonium chloride as a nitrogen source, has the ability to degrade ammonia nitrogen and the ability to degrade total nitrogen in an aerobic denitrification medium. The bacterial strain is suitable for the biological degradation and biological remediation of ammonia nitrogen and total nitrogen in agricultural land wastewater. However, the strain obtained in the invention does not have the activity of degrading 2-aminoethanol (Primary Amines), 2-diethylaminoethanol (Tertiary Amines) and piperazine (Heterocyclic Amines). In addition, the use of the obtained strain in wastewater treatment process, especially since there are no adapted species isolated from that type of wastewater, does not show sufficient activity in water treatment by causing the death of microorganisms depending on the amount of nitrogen in the water content.

The limitations and inadequacies of the solutions in the state of the art, the difficulties in the state of the art such as slow growth of microorganisms and simultaneous nitrification/denitrification conditions in the methods used in the treatment of wastewater, the inadequacy of the activated sludge systems used today in the wastewater treatment process in the presence of recalcitrant compounds, the inadequacy of the microorganisms in the early stages of the process due to their inadequate adaptation levels and therefore their inability to perform their functions, the inability of microorganisms to degrade 2-aminoethanol (Primary Amines), 2-Diethylaminoethanol (Tertiary Amines) and piperazine (Heterocyclic Amines), the fact that although the existing compounds are nitrogen compounds, their removal in normal wastewater treatment plants is difficult due to both toxicity and the fact that they are difficult to decompose, and the fact that the retention time of the wastewater in the treatment plant is extended due to the very long duration of these removal processes, have necessitated a development in the treatment of wastewater.

### Brief Description and Aims of the Invention

The invention describes a bacterial culture mixture that eliminates nitrogen from wastewater in order to biodegrade amine compounds such as 2-aminoethanol (Primary Amines), 2-diethylaminoethanol (Tertiary Amines) and piperazine (Heterocyclic Amines) found in refinery wastewater and the method of obtaining this mixture. In the bacterial culture mixture of the invention, bacteria belonging to the genera *Arthrobacter, Pseudomonas, Pigmentiphaga, Acidovorax, Chitinophaga* obtained as pure isolates from activated sludge are multiplied in 5L bioreactor systems, in appropriate mineral medium, trace element solution and bacterial growth media. With said bacterial culture mixture, 2-aminoethanol, 2-Diethylaminoethanol and piperazine in wastewater are degraded and water is treated.

The aim of the invention is to ensure water treatment by enabling the biodegradation of amine compounds found in refinery wastewater. The bacteria belonging to the genera *Arthrobacter, Pseudomonas, Pigmentiphaga, Acidovorax, Chitinophaga,* which perform nitrogen elimination in the invention, break down 2-aminoethanol, 2-Diethylaminoethanol and piperazine compounds in refined wastewater, thus degrade the amine compounds in the water content and treating the water.

Another aim of the invention is to provide a bacterial culture mixture that quickly and easily adapts to the system to which they are given for the aim of treating refinery wastewater. The bacteria used in the mixture in the invention are isolated from the refinery wastewater system activated sludge, which is their natural habitat, thus the bacteria, which are already accustomed to the environment containing ammonium compounds, show rapid adaptation to the amines present during the treatment of wastewater.

Another aim of the invention is to provide a bacterial culture mixture that has the ability to degrade 2-aminoethanol, 2-Diethylaminoethanol and piperazine compounds with high efficiency. In order to increase the ability of the bacteria in the mixture to degrade said substrates, the organisms are acclimated to the relevant chemicals in advance, ensuring that the organisms adapt to the change in the environment and maintain their performance in the long term.

Another aim of the invention is to provide a bacterial culture mixture that has the ability to eliminate nitrogen in refinery wastewater even in the presence of recalcitrant compounds. In the invention, the degradation of even recalcitrant compounds that cannot be easily degraded microbiologically is carried out by oxidative mechanisms.

### Detailed Description of the Invention

The invention relates to a bacterial culture mixture that eliminates nitrogen from wastewater in order to biodegrade amine compounds found in refinery wastewater and the method of obtaining this mixture. In the bacterial culture mixture of the invention, bacteria belonging to the genera Arthrobacter, *Arthrobacter*, *Pseudomonas*, *Pigmentiphaga*, *Acidovorax*, *Chitinophaga* obtained as pure isolates from refinery wastewater system activated sludge are multiplied in bioreactor systems, preferably in a 5L bioreactor, suitable mineral medium, trace element solution and bacterial growth media. Said bacterial culture mixture contains bacteria belonging to the genera *Arthrobacter, Pseudomonas, Pigmentiphaga, Acidovorax, Chitinophaga* and provides treatment of water by degrading 2-aminoethanol, 2-diethylaminoethanol and piperazine in wastewater.

The bacterial culture mixture, which is the subject of the invention and performs nitrogen elimination to ensure biodegradation of amine compounds, contains pure bacterial isolates of the genera Arthrobacter, Pseudomonas, Pigmentiphaga, Acidovorax, Chitinophaga at a volume ratio of 2-20% (v/v). The bacteria in the mixture are present in equal proportions.

The preparation method of nitrogen eliminating bacterial culture mixture to provide biodegradation of amine compounds, which is the subject of the invention, comprises the process steps of:
i. incubating bacteria belonging to the genera *Arthrobacter*, *Pseudomonas*, *Pigmentiphaga, Acidovorax, Chitinophaga* in standard microbial agar growth medium at 25-30°C for 12-72 hours,
ii. diluting pure bacterial isolates belonging to the genera *Arthrobacter, Pseudomonas, Pigmentiphaga, Acidovorax, Chitinophaga* in physiological saline after incubation, and adjusting their optical densities (OD), and
iii. obtaining a bacterial culture mixture containing 2-20% (v/v) of *Arthrobacter, Pseudomonas, Pigmentiphaga, Acidovorax, Chitinophaga* genera by taking 5-10% by volume (v/v) of the bacterial isolates whose ODs were adjusted and adding them to the liquid medium, which is the bacterial growth media, and growing them at 25-30°C and 50-200 rpm for 12-72 hours.

In the invention, 2-20% by volume (v/v) *Arthrobacter, Pseudomonas, Pigmentiphaga, Acidovorax, Chitinophaga* genera bacteria are isolated from activated sludge as pure isolates and bacteria are grown in the bioreactor in bacterial growth media at a volumetric weight ratio of 0.05%-5%, containing at least one of the salts of potassium dihydrogen phosphate (KH₂PO₄), potassium chloride (KCI), magnesium sulphate (MgSO₄), calcium chloride (CaCl₂), sodium chloride (NaCl), iron(III) chloride (FeCl₃) at a rate of 0.01-1% by weight in volume to provide the basic mineral environment that bacteria will need for reproduction, development and assimilation, and containing, in the presence of distilled water at a rate of 70-95% by volume, at least one of the hydrochloric acid (HCl), manganese sulphate (MnSO₄), boric acid (H₃BO₃), zinc sulphate (ZnSO₄), iron(II) sulphate (FeSO₄), copper(II) sulphate (CuSO₄) compounds at a rate of 0-0.5% by weight in volume as a trace element solution. In addition, optimum conditions are provided by adding yeast, peptone and glucose at a rate of 0.05% - 1% to the growth medium in order to meet the carbon, nitrogen and phosphorus needs of the bacteria. Said bacterial culture mixture is at a rate of 2-20% by volume, and with this rate, the full and sufficient use of the contents in the medium is ensured for each bacterial cell. Said bacterial culture mixture biologically degrades the most common amines in refinery wastewater, 2-aminoethanol, 2-diethylaminoethanol and piperazine, by eliminating nitrogen. In studies conducted using microorganism consortium, changes in chemical parameters were monitored at certain time intervals. In time and day-dependent experiments, a significant decrease was observed in the level of chemical oxygen demand (COD), which expresses the total organic load. At the same time, it was determined that total Kjeldahl nitrogen (TKN) levels decreased over time, but the ammonium nitrogen level remained constant. This situation indicates that organic nitrogen-containing amine compounds are degraded as a result of microbial activity. The experimental results show that the consortium can effectively degrade organic pollutants. With this decomposition, said amines are treated from wastewater.

In order to increase the ability of the bacteria in the mixture to degrade asid 2-aminoethanol, 2-diethylaminoethanol and piperazine substrates, the organisms are pre-acclimatised against the relevant chemicals. The acclimation process is a pre-adaptation process in which the growth media is added in a way that will have low ppm concentrations of the relevant chemicals, and the gene regions that synthesise the relevant degradation enzymes of the bacteria are activated and adapted.

Since the content of the growth medium components and the optimum incubation condition for each isolate in the distilled water at a volume of 5-10% by weight are different in bioreactors, inoculation was made at an average of 10-10¹⁰ cfu/mL, and aerobically propagated for approximately 12-72 hours at 25°C-30°C, 6-8 pH, 50-200 rpm according to the growth curve results (the hours at which microorganisms that degrade amine compounds pass into the logarithmic phase varied between 5 and 24 hours for each isolate). These conditions are specifically selected to support the optimal growth and metabolic activities of the organism. Said conditions provide the environment in which the organism operates at the highest efficiency. A change in the temperature, pH or stirring speed used outside the specified conditions may slow down the growth rate of the organism, reduce the production of metabolic products or cause the formation of undesirable by-products. This leads to a decrease in the technical efficiency. Each isolate inoculated into the bioreactor with an optical density of 10-10¹⁰ cfu/mL was cultured using the pour plate method during production, their viability was determined and the final concentration range was checked. In the pour plate method, one mL of bacterial dilutions is transferred to sterile petri dishes; agar medium cooled to 45°C is poured on top and mixed. In addition, the total viability of the microorganisms grown after production was counted. The isolates reach 10⁸-10⁹ cfu/mL in an average of 12-72 hours. The isolate bacteria are then applied by adding them to water containing real wastewater. Research conducted to date has shown that the degradation of amines by aerobic bacteria is via oxidative mechanisms. Therefore, the bacteria isolated in the invention also perform the degradation of amines via oxidative mechanisms. In the invention, media, trace solution, mineral medium and bacteria are all present in this culture mixture.

### REFERENCES

[1]Wastewater treatment water use completed. Wastewater Treatment Water Use | U.S. Geological Survey. (n.d.). Retrieved December 14, 2022, from https://www.usgs.gov/special-topics/water-science-school/science/wastewater-treatment-water-use
[2]The importance of wastewater treatment. VWR. (2021, May 26). Retrieved December 14, 2022, from https://us.vwr.com/cms/tech-article-importance-of-wastewater-treatment
[3]Handling of amine-based wastewater produced during Carbon Capture. (n.d.). Retrieved December 14, 2022, from https://www.researchgate.net/publication/338224640_Handling_of_Amine-based_Wastewater_Produced_During_Carbon_Capture
[4]Nitrogen and water completed. Nitrogen and Water | U.S. Geological Survey. (n.d.). Retrieved December 14, 2022, from https://www.usgs.gov/special-topics/water-science-school/science/nitrogen-and-water
[5]Ma B, Wang S, Cao S, Miao Y, Jia F, Du R, Peg Y. "Biological nitrogen removal from sewage via anammox: Recent advances". Bioresource Technology, Vol. 200, Jan 2016, pages 981-990.
[6]What is activated sludge? | water & wastes digest. (n.d.). Retrieved December 14, 2022, from https://www.wwdmag.com/biosolids-management/sludge-dewatering/article/10939479/what-is-activated-sludge

## Claims

1. A mixture of nitrogen eliminating bacterial cultures to ensure biodegradation of amine compounds, comprising pure bacterial isolates of the genera *Arthrobacter, Pseudomonas, Pigmentiphaga, Acidovorax, Chitinophaga* at 2-20% (v/v) by volume.

2. A mixture of bacterial cultures according to Claim 1, wherein the amine compounds that are biodegraded are 2-aminoethanol, 2-diethylaminoethanol or piperazine.

3. A mixture of bacterial cultures according to Claim 1 or Claim 2, wherein said bacterial isolates are present in equal proportions.

4. The preparation method of nitrogen eliminating bacterial culture mixture to provide biodegradation of amine compounds, comprising the process steps of:
**i.** incubating bacteria belonging to the genera Arthrobacter, Pseudomonas, Pigmentiphaga, Acidovorax, Chitinophaga in standard microbial agar growth medium at 25-30°C for 12-72 hours,
**ii.** diluting pure bacterial isolates belonging to the genera Arthrobacter, Pseudomonas, Pigmentiphaga, Acidovorax, Chitinophaga in physiological saline after incubation, and adjusting their optical densities (OD), and
**iii.** obtaining a bacterial culture mixture containing 2-20% (v/v) of *Arthrobacter, Pseudomonas, Pigmentiphaga, Acidovorax, Chitinophaga* genera by taking 5-10% by volume (v/v) of the bacterial isolates whose ODs were adjusted and adding them to the liquid medium, which is the bacterial growth media, and growing them at 25-30°C and 50-200 rpm for 12-72 hours.

5. A bacterial culture mixture prepared by a method according to claim 4.
